# EUROPÄISCHE PATENTSCHRIFT

(11) **EP 1 935 871 B1**
(45) Veröffentlichungstag und Bekanntmachung des Hinweises auf die Patenterteilung: **08.07.2015**
(21) Anmeldenummer: 07023804.3
(22) Anmeldetag: 08.12.2007
(51) Int. Cl.: C07C 209/36, C07C 211/50

(54) **Verfahren zur Herstellung von aromatischen Aminen**
Method for manufacturing aromatic amines
Procédé destiné à la fabrication d'amines aromatiques

(30) Priorität: 18.12.2006 DE 102006059678
(43) Veröffentlichungstag der Anmeldung: 25.06.2008
(73) Patentinhaber: Bayer Intellectual Property GmbH, 40789 Monheim (DE)
(72) Erfinder: Pennemann, Bernd, Dr., 51467 Bergisch Gladbach (DE); Brady, Bill, Dr., 77053 Houston Texas (DE); Buse, Rainer, Dr., 51061 Köln (DE)
(74) Vertreter: BIP Patents

(56) Entgegenhaltungen:
- EP-A- 0 223 035
- EP-A- 1 602 640
- US-B1- 6 472 564

## Beschreibung

Die Erfindung betrifft ein Verfahren zur katalytischen Hydrierung von Nitroaromaten und der destillativen Aufarbeitung der dabei anfallenden wässrigen Aminlösungen. Bei diesem Verfahren kann energieeffizient sowohl das Amin von Wasser weitgehend befreit als auch das Wasser Amin- und Leichtsieder-frei erhalten als auch die Leichtsieder gewonnen werden.

Es ist beispielsweise aus EP-A-0 223 035 bekannt, dass man aromatische Diamine wie z.B. Toluylendiamin (TDA, Diaminotoluol) durch katalytische Hydrierung der entsprechenden aromatischen Dinitroverbindungen herstellen kann. Die Hydrierung kann unter Mitverwendung von Lösungsmitteln wie beispielsweise niedrig siedenden Alkoholen wie Methanol, Ethanol oder Isopropanol erfolgen. Die Hydrierung wird mit Hilfe von im Reaktionsgemisch dispergierten Katalysatoren durchgeführt, die dann durch Filtration oder Sedimentation abgetrennt und gegebenenfalls in den Prozess zurückgeführt werden. Die Hydrierreaktion ist dabei sehr stark exotherm. Ständige Aufgabe bei der Hydrierung beispielsweise von Dinitrotoluol (DNT) zu Toluylendiamin (TDA) ist es daher, diese Wärme nicht als Abwärme abzuführen sondern sinnvoll zu nutzen. So beschreibt WO-A-96/11052 eine Reaktionsapparatur für die Durchführung von Schlammphasenhydrierungen unter Verwendung der Reaktionswärme für die Erzeugung von nutzbarem Dampf. Wesentlich für die Nutzbarkeit des Dampfes ist jedoch eine ausreichend hohe Temperatur, die wiederum eine entsprechend hohe Reaktionstemperatur bedingt.

So wird in EP-A-0223035 beispielsweise Dampf in einem Bereich von 5 bis 30 bar erzeugt. Unter Berücksichtigung der üblichen Temperaturgradienten in Wärmetauschern ist für die Erzeugung derartigen Dampfes eine Reaktionstemperatur von 160°C oder höher erforderlich. In EP-A-0223035 werden Temperaturen im Bereich von 170 bis 250°C genannt.

Bislang erfolgte die Aufarbeitung des bei der Hydrierung anfallenden Reaktionsgemisches dergestalt, dass man ein nach Abtrennung des gegebenenfalls mit verwendeten Lösungsmittels vorliegendes Gemisch aus aromatischen Diaminen und Reaktionswasser zunächst kontinuierlich unter Normal- oder Überdruck in einer Destillationskolonne von Wasser befreit und anschließend das als Destillationsrückstand anfallende Diamin gegebenenfalls in weiteren Verfahrensschritten von noch anhaftendem Wasser und von gegebenenfalls noch vorliegenden organischen Verbindungen befreit. Bei dieser Arbeitsweise entstehen als Destillate stets Gemische von Wasser mit wasserdampfflüchtigen organischen Nebenprodukten, wie sie bei der Hydrierung von Dinitroaromaten anfallen. Es handelt sich bei diesen Nebenprodukten beispielsweise um aromatische oder cycloaliphatische Alkohole, d.h. beispielsweise im Falle der Herstellung von Diaminotoluol um Toluidine, Methylcyclohexylamine und Methylcyclohexyldiamine und/oder Methylcyclohexanol.

Diese wasserdampfflüchtigen Nebenprodukte bewirken, dass das über Kopf destillierte Wasser stark mit diesen Verbindungen belastet ist. In EP-A-0236839 ist ein Verfahren zur destillativen Aufarbeitung von derartigen wässrigen Aminlösungen beschrieben, bei welchem ein weit weniger mit organischen Verunreinigungen belastetes Abwasser anfällt. Dazu wird das Gemisch in einer Destillationskolonne mit Seitenentnahme aufgetrennt. Die Brüden der Destillationskolonne werden kondensiert, die hierbei anfallende flüssige Phase über eine Phasentrennapparatur geführt, in welcher dem Brüdenkondensat wasserdampfflüchtige organische Nebenprodukte als organische Phase entnommen werden. Die wässrige Phase wird auf den Kopf der Destillationskolonne zurückgeführt. Das weitgehend von wasserdampfflüchtigen organischen Verunreinigungen befreite Wasser wird über einen Seitenstrom entnommen. Die von Wasser und wasserdampfflüchtigen Verunreinigungen befreiten Diamine fallen dabei als Sumpfprodukt an.

US6472564 beschreibt ein Verfahren zur Herstellung von aromatischen Aminen, wobei die Destillation des Reaktionsgemisch durch zwei Kolonnen getrieben wird, wobei die erste Kolonne unter atmosphärischem Druck, und die zweite Kolonne mit einem absoluten Kopfdruck von 100 mbar betrieben wird.

All diesen oben genannten Verfahren ist gemeinsam, dass die destillative Abtrennung des Wassers aus dem bei der Hydrierung enthaltenden Reaktionsgemisches enthaltend TDA und Wasser bei Normal- oder Überdruck durchgeführt wird, so dass die bei der Hydrierung anfallende Wärme aufgrund des Temperaturniveaus des erzeugten Dampfes für die Destillation des Reaktionsgemisches enthaltend TDA und Wasser nicht in wesentlichem Umfang genutzt werden kann. Daher weisen die oben genannten Verfahren einen hohen Energieverbrauch in Form von Heizdampf auf: pro kg abzutrennendem Wasser müssen 1,2 bis 2 kg Heizdampf eingesetzt werden.

Ein Verfahren, das mit 30-50% weniger Heizdampf auskommt als die anderen Verfahren des Standes der Technik wird in EP-A-1602640 beschrieben. Hierbei wird eine zwei- oder mehrstufige Kolonnenanordnung gewählt, deren Drücke und Temperaturen so gewählt werden, dass mit der bei der Kondensation des Brüdenstroms einer Kolonne freiwerdenden Wärme der Verdampfer der davor oder dahinter angeordneten Kolonne betrieben werden kann. Beispielsweise wird eine Anordnung von zwei Kolonnen dargestellt, bei der die erste mit einem absoluten Kopfdruck von 0,6 bar und die zweite mit einem absoluten Kopfdruck von 3 bar betrieben wird, wobei der Verdampfer der ersten Kolonne mit den Brüden der zweiten Kolonne beheizt wird. Ein großer Nachteil dieses Verfahrens sind jedoch der erhöhte Investitionsaufwand für eine zusätzliche Kolonne und die für den Betrieb erforderlichen Pumpen, Rohrleitungen, Instrumenten usw., durch die die Wirtschaftlichkeit des Verfahrens reduziert wird. Der Verbrauch an Heizdampf pro kg abzutrennendem Wasser liegt entsprechend bei ca. 0,7 bis 1,4 kg Heizdampf.

Die Aufgabe der vorliegenden Erfindung besteht daher darin, ein einfaches und wirtschaftliches Verfahren zur Herstellung und destillativen Aufarbeitung von Aminen zu finden, das mit geringem Einsatz an zugeführter Energie arbeitet.

Die Erfindung betrifft ein Verfahren zur Herstellung von aromatischen Aminen durch katalytische Hydrierung der entsprechenden Nitroaromaten und deren destillative Reinigung, bei dem
a) die Nitroaromaten in der flüssigen Phase in einem Reaktionsapparat in Gegenwart eines Katalysators bei Temperaturen von 100 bis 200°C hydriert werden und Drucken von 5 bis 100 bar, wobei ein Reaktionsgemisch enthaltend Amin und Wasser erhalten wird, und
b) aus dem Reaktionsgemisch enthaltend Amin und Wasser das Wasser destillativ abgetrennt wird und dabei in einem einzigen Destillationsschritt ein gereinigtes Amin enthaltend Wasser in Gehalten von weniger als 20 Gew.%, bezogen auf das Gewicht des Gemisches, erhalten wird, und
c) die Reaktionswärme der Umsetzung in Schritt a) zumindest teilweise zur Beheizung des in Schritt b) durchgeführten Destillationsschritts genutzt wird, und
d) die Destillationskolonne mit einem absoluten Kopfdruck von 0.3 bis 0.8 bar betrieben wird.

Die Hydrierung in Schritt a) wird bei Temperaturen von 100 bis 200°C, bevorzugt von 120 bis 180°C, besonders bevorzugt von 125 bis 170°C und ganz besonders bevorzugt von 130 bis 160°C in Gegenwart von Katalysatoren bei Drücken von 5 bis 100, bevorzugt von 8 bis 50 und besonders bevorzugt von 10 bis 35 bar durchgeführt.

Als Nitroaromaten können im Prinzip alle technisch übliche Nitroaromaten eingesetzt werden. Bevorzugt werden aromatische Mono- und / oder Dinitroverbindungen eingesetzt. Besonders bevorzugt werden Nitrobenzol, Nitrotoluol und Dinitrotoluol eingesetzt.

Als Reaktionsapparat kann beispielsweise der in WO-A-96/11052 beschriebene Schlammphasenreaktor eingesetzt werden. Geeignete andere Reaktoren sind z.B. in EP-A-0236 935 oder US-A-6350911 beschrieben. Selbstverständlich können auch mehrere gleiche oder Kombinationen unterschiedlicher geeigneter Reaktionsapparate eingesetzt werden.

Als Katalysatoren können alle zur katalytischen Hydrierung von aromatischen Nitroverbindungen bekannten Hydrierkatalysatoren eingesetzt werden. Gut geeignet sind insbesondere die Metalle der 8. Nebengruppe des Periodensystems der Elemente oder Mischungen derselben, die beispielsweise auf Trägermaterialien wie Kohlenstoff oder Oxiden von Magnesium, Aluminium und/oder Silizium aufgebracht sein können. Vorzugsweise werden Raney-Eisen, -Kobalt und/oder -Nickel, insbesondere Nickel-haltige Katalysatoren wie beispielsweise Raney-Nickel-Katalysatoren sowie Palladium oder Platin-haltige Katalysatoren auf Trägermaterialien eingesetzt, deren Herstellung und Anwendung als Hydrierkatalysator von aromatischen Nitroverbindungen wie z.B. Nitrobenzol, Nitrotoluolen, Dinitrotoluolen, chlorierten Nitroaromaten und anderen bekannt ist und bereits öfters beschrieben wurde (z.B. EP-A 0223035, EP-B-1066111, EP-A-1 512 459).

Die Nutzung der Reaktionswärme in Schritt c) kann entweder direkt oder indirekt erfolgen. Die Nutzung der Reaktionswärme erfolgt bevorzugt in einem Wärmeaustauscher, der in dem Reaktor selbst angeordnet sein kann (beispielsweise im Inneren des Reaktors oder als Kühlmantel) oder der mit dem Reaktor verbunden ist (beispielsweise als externer Wärmeaustauscher, der mit dem Kühlmedium des Reaktors oder dem umgepumpten Reaktorinhalt selbst beheizt wird). Bei der direkten Nutzung wird ein Strom aus dem Sumpf der in Schritt b) eingesetzten Destillationskolonne durch den Wärmeaustauscher geführt. Bei der indirekten Nutzung wird ein Wärmeträger, beispielsweise Wasserdampf, verwendet, d.h. die Reaktionswärme wird zunächst zur Erzeugung von beispielsweise Wasserdampf genutzt, und der Wasserdampf anschließend zur Beheizung des Sumpfs der in Schritt b) eingesetzten Destillationskolonne eingesetzt.

Dabei ist zu beachten, dass bei der Hydrierung mehr Reaktionswärme frei wird als für die Beheizung der Destillation erforderlich ist. Der Vorteil der Erzeugung von Wasserdampf besteht dann darin, dass die überschüssige Reaktionswärme auf diese Weise leicht auch anderen Einsatzzwecken zugeführt werden kann. Hierzu gehören beispielsweise die Wasserdampfstrippung von organisch belasteten wässrigen Lösungen oder Säuren, die Vorwärmung von Extraktions- und Destillationszuläufen sowie der Betrieb von Kälteanlagen, beispielsweise Absorptionskältemaschinen sowie zahllose mögliche Formen der Beheizung. Als mögliche Anwendungen seien beispielhaft und ohne Anspruch auf Vollständigkeit genannt: Beheizung von Chemieanlagen, beispielsweise deren Rohrleitungen und Tanklägern, beispielsweise mittels Wärmetauschern, Strippung von Prozesswasser- und Abwasserströmen wie sie bei Hydrierprozessen wie etwa der Anilin- oder TDA-Herstellung anfallen, Strippung von organisch verunreinigten Schwefelsäuren wie bei Nitrierprozessen wie etwa der Nitrobenzol- oder DNT-Herstellung anfallen und die Vorwärmung von Zuläufen zu Anlagen, in denen diese Strippung durchgeführt wird sowie die Erwärmung von Kesselspeisewasser.

Der erzeugte Wasserdampf weist bevorzugt einen absoluten Druck von 1 bis 12 bar, bevorzugt 1,5 bis 8 bar, besonders bevorzugt 2 bis 6 bar und ganz besonders bevorzugt 2 bis 5 bar auf.

Aus dem in der Hydrierung in Schritt a) erhaltenen Reaktionsgemisch enthaltend Amin und Wasser werden Katalysator und gelöste Gase abgetrennt. Das so erhaltene Reaktionsgemisch wird gegebenenfalls noch von Lösungsmittel befreit. Wird eine Abtrennung von Lösungsmittel durchgeführt, so geschieht das wiederum vorteilhaft unter Verwendung der Reaktionswärme der Hydrierung, entweder direkt oder indirekt mittels der Erzeugung von Wasserdampf. Das von Lösungsmittel befreite Reaktionsgemisch enthaltend Amin und Wasser weist üblicherweise eine Aminkonzentration von 50 bis 70 Gew.-%, bevorzugt von 55 bis 65 Gew.-%, bezogen auf das Reaktionsgemisch enthaltend Amin und Wasser auf. Neben den gewünschten Aminverbindungen enthält das Reaktionsgemisch enthaltend Amin und Wasser üblicherweise zusätzlich bis zu 5 Gew.-%, vorzugsweise 500 bis 5000 ppm wasserdampfflüchtige Verunreinigungen der oben genannten Art.

In dem erfindungsgemäßen Verfahren werden wesentliche Teile des Wassers und der wasserdampfflüchtigen Verunreinigungen (beispielsweise aromatische oder cycloaliphatische Alkohole) in Schritt b) von dem Reaktionsgemisch enthaltend Amin und Wasser abgetrennt. Dabei wird gemäß Schritt c) die Reaktionswärme der Umsetzung in Schritt a) zumindest teilweise zur Beheizung des in Schritt b) durchgeführten Destillationsschritts genutzt. Bevorzugt wird mindestens einer der Verdampfer zumindest teilweise mit Wasserdampf beheizt, der unter Nutzung der Reaktionswärme der Umsetzung in Schritt a) erzeugt worden ist. Das bei der Destillation in Schritt b) in einem einzigen Destillationsschritt erhaltene Sumpfprodukt enthält weniger als 20 Gew.%, bevorzugt weniger als 10 Gew.-% und besonders bevorzugt weniger als 5 Gew.-% Wasser und ganz besonders bevorzugt weniger als 3 Gew.-% Wasser, bezogen auf das Gewicht des gereinigten Amins enthaltend Wasser.

Bevorzugt werden so mindestens 50%, besonders bevorzugt mindestens 60% des Energiebedarfs der Destillation in Schritt b) durch die Beheizung unter Nutzung der Reaktionswärme der Umsetzung in Schritt a) gedeckt. Besonders bevorzugt werden mindestens 50%, besonders bevorzugt mindestens 60% des Energiebedarfs der Destillation in Schritt b) durch den unter Nutzung der Reaktionswärme der Umsetzung in Schritt a) erzeugten Wasserdampf gedeckt und so der Einsatz von zusätzlichem Frischdampf deutlich reduziert. Dies wird möglich durch die Destillation bei einem absoluten Kopfdruck von unter 1 bar und das daraus resultierende niedrige Temperaturniveau bei der Destillation.

In dem einen einzigen Destillationsschritt in Schritt b) wird also das Wasser aus dem Reaktionsgemisch enthaltend Amin und Wasser, das üblicherweise Aminkonzentration von 55 bis 70 Gew.-% und Wasserkonzentrationen von 30 bis 45 Gew.-%, bezogen auf das Gewicht des Reaktionsgemisches enthaltend Amin und Wasser, aufweist, so weitgehend entfernt, dass sich die oben genannten Wassergehalte ergeben. Das schließt aber nicht aus, dass beispielsweise bei der vorher ggf. stattfinden Lösungsmittelabtrennung aus dem Reaktionsgemisch enthaltend Amin und Wasser auch bereits Anteile von Wasser mit abgetrennt werden oder dass in im Anschluss durchgeführten weiteren Destillationsschritten noch Wasser aus dem gereinigten Amin entfernt wird.

Der Verdampfer ist dabei keinen Beschränkungen unterworfen, es können beispielsweise die in der Technik üblichen Zwangs- oder Naturumlaufverdampfer oder sich innerhalb der Kolonne befindliche interne oder externe Heizbündel eingesetzt werden. Insbesondere können auch Kombinationen mehrerer gleicher oder unterschiedlicher Verdampfer eingesetzt werden. In diesem Falle ist mindestens einer der Verdampfer mit der Wärme der Hydrierreaktion zu betreiben und dabei bevorzugt so auszulegen, dass diese mindestens 50% der Gesamtleistung der Verdampfer dieses Destillationsschrittes erbringt. In einer weiteren Ausführungsform des erfindungsgemäßen Verfahrens wird der aus der Kolonne austretende Sumpfstrom ganz oder teilweise durch den mit dem Hydrierreaktor verbundenen Wärmeaustauscher geführt und durch die Reaktionswärme direkt beheizt. Die Kolonne kann beispielsweise als Glockenboden-, Packungs- oder Füllkörperkolonne ausgeführt werden, wobei die Kolonne 12 bis 50, bevorzugt 20 bis 40 theoretische Trennstufen aufweist. Die Kolonne wird mit einem absoluten Kopfdruck von weniger als 1 bar, bevorzugt 0,3 bis 0,8 bar betrieben. Das zu trennende Reaktionsgemisch enthaltend Amin und Wasser wird bevorzugt oberhalb des Verdampfers, besonders bevorzugt zwischen der zweiten und der achten theoretischen Trennstufe aufgegeben. Die Brüden der Kolonne werden kondensiert und teilweise als Rücklauf auf die Kolonne gegeben, wobei das Rücklaufverhältnis vorzugsweise mindestens 0,2, besonders bevorzugt 0,3 bis 0,6 beträgt.

In einer weiteren erfindungsgemäßen Ausführungsform des Verfahrens wird eine wasserdampfflüchtige organische Phase abgetrennt und die wässrige Phase wieder auf den Kopf der Kolonne gegeben. Die Destillatentnahme, d.h. die Entnahme des Wassers, erfolgt bevorzugt über einen Seitenstrom, der mindestens 4, vorzugsweise 5 bis 15 theoretische Trennstufen unterhalb des Kopfes der Kolonne und mindestens 8, vorzugsweise 15 bis 25 theoretische Trennstufen oberhalb des Sumpfes der Kolonne angeordnet ist. Hierbei liegt das Volumenverhältnis von Rücklauf (unterhalb der Entnahmestelle) zu der Entnahme des Wassers vorzugsweise bei mindestens 0,2, besonders bevorzugt bei 0,3 bis 0,6.

Der große Vorteil des erfindungsgemäßen Verfahrens liegt also darin, dass die bei der Hydrierung auch bei niedrigen Temperaturen anfallende Reaktionswärme für die Abtrennung von Wasser aus dem erzeugten Reaktionsgemisch enthaltend Amin und Wasser eingesetzt werden kann. Dies wird ermöglicht durch die Destillation bei absoluten Drücken von < 1 bar, bei der in nur einem einzigen Schritt das Wasser aus dem TDA weitgehend entfernt wird. Dadurch wird es möglich, wie anhand des Beispiels dargestellt, die Destillation mit Fremddampfmengen von weniger als 0,6 kg Dampf pro kg abgetrennten Wassers (im Beispiel: 0,45 kg Heizdampf pro kg abgetrennten Wassers) durchzuführen. Mit Fremddampf ist dabei Dampf gemeint, der nicht aus der Reaktionswärme der Hydrierung gewonnen wird. Dies stellt gegenüber dem Stand der Technik eine Einsparung von mehr als 50% dar.

Bei den Verfahren nach dem Stand der Technik war das nicht möglich, da die destillative Abtrennung von Wasser dort üblicherweise bei Normal- oder Überdruck und somit bei hohen Temperaturen durchgeführt wird und das Temperaturniveau des bei niedrigen Hydriertemperaturen von 100-200°C erzeugten Wasserdampfs dann nicht ausreicht.

In dem einzigen bekannten Verfahren, in dem Unterdruck für die Destillation eingesetzt wird, ist nach der Lehre von EP-A-1602640 ein Verbund von mehreren, untereinander verbundenen Destillationskolonnen notwendig, die jedoch ebenfalls ohne Nutzung der Wärme aus der Hydrierung betrieben werden. Denn nach der Lehre von EP-A-1602640 wird der Verdampfer der bei niedrigen Drücken und damit auch niedrigen Temperaturen betriebenen Kolonne mit den Brüden aus der bei höheren Drücken und somit höheren Temperaturen betrieben Kolonne beheizt, so dass gar keine Möglichkeit für die Nutzung der Hydrierwärme in einer bei Unterdruck betriebenen Destillationskolonne besteht.

### Beispiel

In einem Reaktor wird ein DNT-Isomerengemisch bei 130°C und einem Druck von 22 bar hydriert. Die Reaktionswärme wird über einen innenliegenden Wärmetauscher abgeführt, in dem Wasser bei einem Druck von 1,5 bar absolut verdampft wird. Das Reaktionsgemisch wird filtriert, auf Normaldruck entspannt. Es handelt sich bei dem Gemisch um eine ca. 60 Gew.%ige Lösung (bezogen auf das Gewicht des Reaktionsgemisches) eines Diamingemisches, bestehend im Wesentlichen aus 77,2 Gew.-% 2,4-Diaminotoluol, 19,3 Gew.-% 2,6-Diaminotoluol und 3,5 Gew.-% anderer Diaminotoluol-Isomeren, jeweils bezogen auf die Summe der Gewichte der Diaminotoluol-Isomeren. Die Lösung weist einen Gehalt an wasserdampfflüchtigen organischen Nebenprodukten von 0,3 Gew.-% auf. Der Wassergehalt der Lösung liegt entsprechend bei ca. 39,7 Gew.%, bezogen auf das Gewicht des Reaktionsgemisches. Das Gemisch wird anschließend in einer Kolonne mit 30 Stufen destilliert. Die Aufgabe des Zulaufs erfolgt auf der 5. Stufe oberhalb des Sumpfes. Die Kolonne wird bei einem absoluten Kopfdruck von 400 mbar betrieben. Die Kolonne hat zwei Verdampfer, wobei der Sumpf der Kolonne in den ersten Verdampfer abläuft. Ein Teil des Sumpfstroms wird in dem ersten Verdampfer verdampft und in die Kolonne zurückgeführt, der andere Teil strömt über einen Überlauf in den zweiten Verdampfer. Ein Teil des in den zweiten Verdampfer übergelaufenen Stroms wird in dem zweiten Verdampfer verdampft und in die Kolonne zurückgeführt, der andere Teil wird als Produktstrom (gereinigtes Amin) abgezogen. Der erste Verdampfer wird mit dem Dampf von 1,5 bar aus der Hydrierreaktion betrieben. Der zweite Verdampfer wird mit Dampf aus einer anderen Quelle betrieben, die Temperatur dieses Dampfes beträgt 170°C. Die Temperatur im Ablauf des ersten Verdampfers beträgt 90°, der Wassergehalt ca. 19,5 Gew.%. Der zweite Verdampfer wird mit einer Ablauftemperatur von 120°C betrieben, wobei sich ein Wassergehalt von 2 Gew.-% einstellt. Der aus dem zweiten Verdampfer abgezogene Produktstrom (gereinigtes Amin) enthält entsprechend ebenfalls Wasser in Konzentrationen von 2 Gew.-%. Das Rücklaufverhältnis am Kopf der Kolonne beträgt 0,5. Der Anteil der über den ersten Verdampfer eingebrachten Hydrierwärme an der insgesamt für die Abtrennung in beiden Verdampfern erforderlichen Energie beträgt 72%.

Der Bedarf an Fremddampf für die Abtrennung in der eingesetzten Kolonne beträgt nur 0,45 kg pro kg abgetriebenes Wasser.

## Patentansprüche

1. Verfahren zur Herstellung von aromatischen Aminen durch katalytische Hydrierung der entsprechenden Nitroaromaten und deren destillative Reinigung, bei dem
a) die Nitroaromaten in der flüssigen Phase in einem Reaktionsapparat in Gegenwart eines Katalysators bei Temperaturen von 100 bis 200°C und Drücken von 5 bis 100 bar hydriert werden, wobei ein Reaktionsgemisch enthaltend Amin und Wasser erhalten wird, und
b) aus dem Reaktionsgemisch enthaltend Amin und Wasser das Wasser destillativ abgetrennt wird und dabei in einem einzigen Destillationsschritt ein gereinigtes Amin enthaltend Wasser in Gehalten von weniger als 20 Gew.%, bezogen auf das Gewicht des Gemisches, erhalten wird, und
c) die Reaktionswärme der Umsetzung in Schritt a) zumindest teilweise zur Beheizung des in Schritt b) durchgeführten Destillationsschritts genutzt wird, und
d) die Destillationskolonne mit einem absoluten Kopfdruck von 0.3 bis 0.8 bar betrieben wird.

2. Verfahren nach Anspruch 1, bei dem in Schritt c) unter Nutzung der Reaktionswärme der Umsetzung in Schritt a) zunächst Wasserdampf erzeugt wird, der dann zur Beheizung des in Schritt b) durchgeführte Destillationsschritts genutzt wird.

3. Verfahren nach Anspruch 2, bei dem der erzeugte Wasserdampf einen absoluten Druck von 1 bis 12 bar aufweist.

4. Verfahren nach einem der Ansprüche 1 bis 3, bei dem als Nitroaromaten Nitrobenzol, Nitrotoluol oder Dinitrotoluol eingesetzt werden.

5. Verfahren nach einem der Ansprüche 1 bis 4, bei dem das Wasser in dem einzigen Destillationsschritt als Seitenstrom entnommen wird.

6. Verfahren nach einem der Ansprüche 1 bis 5, bei dem mindestens 50% des Energiebedarfs der Destillation in Schritt b) durch die Beheizung unter Nutzung der Reaktionswärme der Umsetzung in Schritt a) gedeckt werden.

7. Verfahren nach Anspruch 2, bei dem mindestens 50% des Energiebedarfs der.Destillation in Schritt b) durch die Nutzung des erzeugten Wasserdampfs gedeckt werden.

## Claims

1. Process for preparing aromatic amines by catalytic hydrogenation of the corresponding nitroaromatics and distillative purification thereof, in which
a) the nitroaromatics are hydrogenated in the liquid phase in a reaction apparatus in the presence of a catalyst at temperatures of 100 to 200°C and pressures of 5 to 100 bar to obtain a reaction mixture comprising amine and water, and
b) the water is separated by distillation from the reaction mixture comprising amine and water to obtain, in a single distillation step, a purified amine containing water in contents of less than 20% by weight, based on the weight of the mixture, and
c) the heat of reaction from the conversion in step a) is utilized at least partly for heating of the distillation step conducted in step b), and
d) the distillation column is operated with an absolute top pressure of 0.3 to 0.8 bar.

2. Process according to Claim 1, in which the heat of reaction of the conversion in step a) is first utilized in step c) to raise steam which is then utilized to heat the distillation step conducted in step b).

3. Process according to Claim 2, in which the steam raised has an absolute pressure of 1 to 12 bar.

4. Process according to any of Claims 1 to 3, in which the nitroaromatics used are nitrobenzene, nitrotoluene or dinitrotoluene.

5. Process according to any of Claims 1 to 4, in which the water is withdrawn as a side stream in the sole distillation step.

6. Process according to any of Claims 1 to 5, in which at least 50% of the energy requirement of the distillation in step b) is covered by the heating with utilization of the heat of reaction of the conversion in step a).

7. Process according to Claim 2, in which at least 50% of the energy requirement of the distillation in step b) is covered by the utilization of the steam raised.

## Revendications

1. Procédé de fabrication d'amines aromatiques par hydrogénation catalytique des composés nitroaromatiques correspondants et purification par distillation, dans lequel
a) les composés nitroaromatiques sont hydrogénés en phase liquide dans un appareil de réaction en présence d'un catalyseur à des températures de 100 à 200 °C et des pressions de 5 à 100 bar, un mélange réactionnel contenant une amine et de l'eau étant obtenu, et
b) l'eau est séparée par distillation du mélange réactionnel contenant une amine et de l'eau, et une amine purifiée contenant de l'eau en des teneurs de moins de 20 % en poids, par rapport au poids du mélange, est ainsi obtenue en une étape de distillation unique, et
c) la chaleur de réaction de la réaction à l'étape a) est utilisée au moins en partie pour le chauffage de l'étape de distillation réalisée à l'étape b), et
d) la colonne de distillation est exploitée à une pression de tête absolue de 0,3 à 0,8 bar.

2. Procédé selon la revendication 1, dans lequel de la vapeur d'eau est tout d'abord générée à l'étape c) en utilisant la chaleur de réaction de la réaction à l'étape a), et cette vapeur d'eau est ensuite utilisée pour le chauffage de l'étape de distillation réalisée à l'étape b).

3. Procédé selon la revendication 2, dans lequel la vapeur d'eau générée présente une pression absolue de 1 à 12 bar.

4. Procédé selon l'une quelconque des revendications 1 à 3, dans lequel du nitrobenzène, du nitrotoluène ou du dinitrotoluène est utilisé en tant que composé nitroaromatique.

5. Procédé selon l'une quelconque des revendications 1 à 4, dans lequel l'eau est soutirée en tant que courant latéral lors de l'étape de distillation unique.

6. Procédé selon l'une quelconque des revendications 1 à 5, dans lequel au moins 50 % de la demande énergétique de la distillation à l'étape b) est couvert par le chauffage utilisant la chaleur de réaction de la réaction à l'étape a).

7. Procédé selon la revendication 2, dans lequel au moins 50 % de la demande énergétique de la distillation à l'étape b) est couvert par l'utilisation de la vapeur d'eau générée.
